# EUROPÄISCHE PATENTSCHRIFT

(11) **EP 4 232 209 B1**
(45) Veröffentlichungstag und Bekanntmachung des Hinweises auf die Patenterteilung: **14.05.2025**
(21) Anmeldenummer: 21815898.8
(22) Anmeldetag: 15.10.2021
(51) Int. Cl.: B05B 16/60, C25D 13/22, G01N 17/00, C12Q 1/02

(54) **VERFAHREN ZUM BETREIBEN EINER BEHANDLUNGSANLAGE SOWIE BEHANDLUNGSANLAGE**
METHOD FOR CONTROLLING A COATING PLANT AS WELL AS COATING PLANT
PROCÉDÉ DE CONTRÔLE D'UNE INSTALLATION DE REVÊTEMENT AINSI QU'INSTALLATION DE REVÊTEMENT

(30) Priorität: 22.10.2020 DE 102020127893
(43) Veröffentlichungstag der Anmeldung: 30.08.2023
(73) Patentinhaber: Dürr Systems AG, 74321 Bietigheim-Bissingen (DE)
(72) Erfinder: FREY, Hans-Ulrich, 70734 Fellbach (DE); WESCHKE, Jürgen, 71263 Weil der Stadt (DE)
(74) Vertreter: RPK Patentanwälte Reinhardt und Kaufmann Partnerschaft mbB
(86) Internationale Anmeldenummer: PCT/DE2021/100835
(87) Internationale Veröffentlichungsnummer: WO 2022/083826

(56) Entgegenhaltungen:
- US-A1- 2002 148 738

## Beschreibung

Die Erfindung betrifft ein Verfahren zum Betreiben einer Behandlungsanlage sowie eine Behandlungsanlage.

### Stand der Technik

In Behandlungsanlagen von Betrieben der Oberflächentechnik, beispielsweise Vorbehandlungsanlagen und kathodischen Tauchlackieranlagen (KTL-Anlagen), in denen metallische und nichtmetallische Werkstoffe mit einer Oberflächenbeschichtung versehen werden, wie auch in diversen Zuluftanlagen von z.B. Spritzkabinen wurden in der Vergangenheit bakterizid wirkende Chemikalien und Betriebsstoffe eingesetzt. Durch die Verschärfung der Umweltgesetzgebung wurden diese keimtötend wirkenden Stoffe (z.B. Blei, Chrom, etc.) sukzessive durch umweltverträglichere Stoffe ersetzt. Allerdings führte dies unter den Betriebsbedingungen der Zuluft-, Vorbehandlungs- und KTL-Anlagen zu stark erhöhtem Keim- und Pilzwachstum durch die in den Anlagen vorhandenen wässrigen Medien, Temperaturen zwischen ca. 20°C und 55°C, ausreichendem Nährstoffeintrag durch die zu behandelnden Werkstücke, etc).

Um Prozessbehälter und Spülbehälter, wie auch die zugehörigen Vorlagebehälter, Zuluftanlagen etc., sicher betreiben zu können, werden täglich Proben aus den entsprechenden Bädern und Behältern entnommen und auf ihre bakteriologische Belastung untersucht.

Abhängig von der festgestellten Keimbelastung erfolgt in regelmäßigen oder unregelmäßigen Abständen eine Behandlung der belasteten Bereiche mit Bioziden, wie z.B. Wasserstoffperoxid, meist in Form einer Schockdosierung.

Die Probennahme bedeutet eine relativ große Wartezeit, die, beispielsweise bei hoher Keimbelastung im System, zu kritischen Situationen führen kann, weil einzuleitende Biozidbehandlungen erst mit entsprechender Verzögerung initiiert werden können.

Bekannt ist auch die Möglichkeit, durch eine kontinuierliche Dosierung keimtötender Substanzen die Keimbelastung auf einem akzeptablen Niveau zu halten. Bekannt ist hierzu z.B. das Clorious-Verfahren, bei dem stark oxidatives Chlordioxid produziert und in belastete Bereiche eingegeben wird. Bei einer kontinuierlichen Dosierung besteht jedoch, je nach Verfahren, die Gefahr einer unerwünschten Resistenzbildung.

Aus der US 20020148738 A1 ist ein Verfahren bekannt, mit dem das Vorhandensein biologischer Verunreinigungen in einem Beschichtungsbad oder Reinigungsbad durch Messung des Kohlendioxidgehalts der Atmosphäre über dem Beschichtungsbad und durch Vergleich des gemessenen Kohlendioxidgehalts mit einem Ausgangswert für den Kohlendioxidgehalt bestimmt wird. Wenn der gemessene Kohlendioxidgehalt einen bestimmten Wert erreicht, kann dem betreffenden Bad ein Biozid zugesetzt werden, um den Gehalt an biologischen Verunreinigungen darin zu kontrollieren.

### Offenbarung der Erfindung

Die Aufgabe der Erfindung ist die Schaffung eines Verfahrens zum Betreiben einer Behandlungsanlage, mit dem die Gefahr einer Resistenzbildung vermindert werden kann.

Eine weitere Aufgabe besteht darin, eine Behandlungsanlage zu schaffen, bei der die Gefahr einer Resistenzbildung vermindert ist.

Die Aufgaben werden durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Die in den Patentansprüchen einzeln aufgeführten Merkmale sind in technologisch sinnvoller Weise miteinander kombinierbar und können durch erläuternde Sachverhalte aus der Beschreibung und durch Details aus den Figuren ergänzt werden, wobei weitere Ausführungsvarianten der Erfindung aufgezeigt werden, solange die resultierenden Kombinationen und die weiteren Ausführungsvarianten unter dem Schutzumfang der Ansprüche fallen.

Es wird ein Verfahren vorgeschlagen zum Betreiben einer Behandlungsanlage, mit wenigstens einem Teilbereich, in dem Fluid zur Behandlung von Bauteilen aufbewahrt wird, und/oder eine Behandlung von Bauteilen, insbesondere mit dem Fluid, durchgeführt wird, und/oder ein Fluid und/oder ein Bauteil transportiert wird, wobei wenigstens einer der Teilbereiche durch wenigstens einen Messfühler überwacht wird, der wenigstens eine biologische Komponente zum Nachweis wenigstens einer biologischen Verunreinigungsschicht in Form eines Biofilms als Vorstadium einer Verkeimung, in dem wenigstens einen Teilbereich aufweist. Eine Erfassung und Überwachung der biologischen Verunreinigungsschicht erfolgt von Beginn eines Aufwuchses der biologischen Verunreinigungsschicht auf dem Messfühler an.

Vorteilhaft kann eine Probennahme aus der Behandlungsanlage und damit eine unerwünschte Zeitverzögerung entfallen. Darüber hinaus wird bei der Detektion eines Biofilms bereits in einem Vorstadium eine drohende Verkeimung der Behandlungsanlage erkannt und kann korrigierend eingegriffen werden, bevor biologische schichtartige Verunreinigungen in wässrigen Medien oder feuchten Bereichen der Behandlungsanlage detektiert werden können.

Als Biofilme werde üblicherweise Schichten aus Mikroorganismen und deren Stoffwechselprodukten, so genannten extrazellulären polymeren Substanzen, bezeichnet, die auf feuchten Oberflächen aufwachsen. Typischerweise kommen in nicht-sterilen Umgebungen Mikroorganismen wie beispielsweise Bakterien, Algen, Pilze, Protozoen, vor.

Damit kann ein weiteres Problem des Standes der Technik gelöst werden, das durch die üblichen Probennahmen aus den Prozessbehältern, Spülbehältern und Vorlagebehältern nur die Keimbelastung erfasst wird, die sich im Medium befindet. Durch den Nachweis von Biofilmen kann frühzeitig eine Verkeimungsgefahr erkannt werden, da Biofilme sich an Behälterwandungen und Rohrleitungen befinden und Biofilme erst nach einem gewissen Aufwuchs die Keime in das jeweilige Medium abgeben, die dann in den genommen Proben gemessen werden. Diese Biofilme und ihre Entwicklung können durch die bisher durchgeführte Probennahmepraxis in keiner Weise erfasst werden.

Teilbereiche der mit dem Verfahren betriebenen Behandlungsanlage können beispielsweise umfassen Befeuchter von Zu- und Umluftanlagen von z.B. Spritzkabinen, Hallenbelüftung, etc., Frischwassersysteme; VE-Wassersysteme mit vollensalztem Wasser, hier vor allem ein oder mehrere Ionentauscher und Pufferbehälter, ein oder mehrere Membrananlagen zur Frischwasseraufbereitung und/oder Abwasseraufbereitung, ein oder mehrere Vorbehandlungsanlagen in der Oberflächentechnik (z.B. Automobillackierung), ein oder mehrere kathodische und/oder anodische Tauchlackieranlagen; ein oder mehrere Spritzkabinen mit Nassabscheidung des Overspray.

Anstelle der regelmäßigen Probennahmen können, je nach Erfordernis, ein oder mehrere Messfühler in Form von Biosensoren in die Behälter oder Rohrleitungen der genannten Prozessbehälter, Spülbehälter und/oder Vorlagebehälter eingebaut werden. Diese Biosensoren erfassen und überwachen den Aufwuchs von Biofilmen von Beginn an. Abhängig von den Erfordernissen der jeweiligen Teilbereiche der Behandlungsanlage können individuelle, auf das jeweilige System abgestimmte, Grenzwerte eingestellt werden, die je nach Biofilmdicke zu Alarmmeldungen, Bioziddosierungen oder Änderungen z.B. der Dosiermenge bei kontinuierlichen Bioziddosierungen führen.

Der Messfühler kann vorteilhaft selektiv auf Biofilme und nicht nur auf eine einfache Schmutzschicht oder anorganische Ablagerungen, so genannte Scalingschichten, reagieren, die sich bilden, wenn etwa Löslichkeitsprodukte im Medium als Salze oder dergleichen ausfallen.

Messfühler für die Detektion von Biofilmen sind an sich bekannt. Geeignet sind optische Messverfahren, deren Signale durch Biofilme moduliert werden. Es können dabei physikalische und chemische Parameter detektiert werden und daraus für den Biofilm relevante Daten extrahiert werden, insbesondere mittels rechnergestützten Methoden. Dies kann vorteilhaft in Echtzeit erfolgen.

Gemäß einer günstigen Ausgestaltung kann eine Überwachung des wenigstens einen Teilbereichs durch den wenigstens einen Messfühler kontinuierlich durchgeführt werden.

Dies erlaubt eine frühzeitige Erkennung von möglichen künftigen biologischen schichtartigen Verunreinigungen. Vorteilhaft kann durch Informationen aus einer Datenbank oder dergleichen abgeschätzt oder vorhergesagt werden, wann spätestens eine Biozidbehandlung erforderlich sein könnte. Damit kann eine Unterbrechung im Betrieb der Behandlungsanlage vorausschauend geplant werden.

Durch eine kontinuierliche Onlinemessung in Echtzeit können bisherig notwendige Totzeiten zwischen Probennahme und Vorliegen des Analysenergebnisses entfallen. Kritische Situationen durch ein verunreinigungsbedingtes Umkippen beispielsweise von Behandlungsbädern mit hohen Kostenfolgen können vermieden werden.

Eine Erfassung des Biofilms erfolgt bereits zu Beginn des Aufwuchses auf dem Messfühler und nicht erst dann, wenn Keime in das Prozessmedium und/oder Spülmedium und/oder Medium im Vorlagebehälter abgegeben werden.

Ein frühzeitiger Eingriff zur Reduzierung der Keimbelastung ist möglich und dadurch eine Verringerung der erforderlichen Mengen an Bioziden, um die Systeme stabil zu halten.

Vorteilhaft kann eine Steuerung der Bioziddosierung anhand der Messwerte des Messfühlers bei Überdosierung oder Unterschreitung von festgelegten Grenzwerten erfolgen. Ebenso ist eine unmittelbare Erfassung der Wirksamkeit einer Biozidbehandlung durch Entfernung/Abnahme des aufgewachsenen Biofilms auf dem Messfühler möglich. Vorteilhaft ist eine Erhöhung der Prozessstabilität der Behandlungsanlage möglich. Durch einen geringeren Verbrauch können Kosten für Biozide reduziert werden; ebenso kann die Umweltbelastung durch den geringeren Biozidverbrauch reduziert werden. Günstigerweise ist ein verbesserter Schutz der Mitarbeiter durch eine reduzierte Keimbelastung in der Behandlungsanlage und durch den reduzierten Umgang mit Bioziden möglich.

Gemäß einer günstigen Ausgestaltung kann ein Grenzwert einer biologischen Verunreinigungsschicht zum Nachweis durch den wenigstens einen Messfühler im wenigstens einen Teilbereich abhängig von jeweiligen Prozesserfordernissen in dem wenigstens einen Teilbereich eingestellt werden.

Hierdurch können spezifische Bedingungen eines bestimmten Prozesses berücksichtigt werden.

Gemäß einer günstigen Ausgestaltung kann bei Erfassung eines Messwerts einer biologischen Verunreinigungsschicht durch den wenigstens einen Messfühler im wenigstens einen Teilbereich abhängig von einem vorgegebenen Grenzwert eine Reinigungssequenz in dem wenigstens einen Teilbereich durchgeführt werden. Günstigerweise kann eine Biozidbehandlung auf notwendige Einsätze beschränkt werden.

Gemäß einer günstigen Ausgestaltung kann bei Erfassung eines Messwerts einer biologischen Verunreinigungsschicht durch den wenigstens einen Messfühler im wenigstens einen Teilbereich abhängig von einem vorgegebenen Grenzwert eine Alarmmeldung erfolgen und/oder eine Bioziddosierung in den wenigstens einen Teilbereich hinein erfolgen und/oder bei kontinuierlicher Bioziddosierung eine Anpassung einer Bioziddosierung erfolgen. Hierdurch können spezifische Bedingungen eines bestimmten Prozesses berücksichtigt werden. Ebenso kann der Verbrauch des Biozids gezielt erfolgen und gegebenenfalls beschränkt werden.

Gemäß einer günstigen Ausgestaltung kann eine Position des wenigstens einen Messfühlers in dem wenigstens einen Teilbereich nach einer Wahrscheinlichkeit eines zu erwartenden frühzeitigen und/oder verstärkten Auftretens von biologischen schichtartigen Verunreinigungen ausgewählt werden.

Gemäß einer günstigen Ausgestaltung kann eine Reinigungssequenz bei einer Behandlungsanlage mit mehreren Teilbereichen jeweils für jeden der Teilbereiche einzeln durchgeführt wird.

Gemäß eines weiteren Aspekts der Erfindung wird eine Behandlungsanlage vorgeschlagen mit wenigstens einem Teilbereich zur Durchführung des Verfahrens nach einem der vorhergehenden Ansprüche, wobei wenigstens ein Teilbereich vorgesehen ist, in dem Fluid zur Behandlung von Bauteilen aufbewahrt wird, und/oder eine Behandlung von Bauteilen, insbesondere mit dem Fluid, durchgeführt wird, und/oder ein Fluid und/oder ein Bauteil transportiert wird, wobei wenigstens einer der Teilbereiche wenigstens einen Messfühler aufweist, der wenigstens eine biologische Komponente zum Nachweis wenigstens einer biologischen Verunreinigungsschicht in Form eines Biofilms als Vorstadium einer Verkeimung in dem wenigstens einen Teilbereich aufweist. Die biologische Verunreinigungsschicht ist von Beginn eines Aufwuchses der biologischen Verunreinigungsschicht auf dem Messfühler an erfassbar und überwachbar.

Teilbereiche der Behandlungsanlage können beispielsweise umfassen Befeuchter von Zu- und Umluftanlagen von z.B. Spritzkabinen, Hallenbelüftung, etc., Frischwassersysteme; VE-Wassersysteme mit vollensalztem Wasser, hier vor allem ein oder mehrere Ionentauscher und Pufferbehälter, ein oder mehrere Membrananlagen zur Frischwasseraufbereitung und/oder Abwasseraufbereitung, ein oder mehrere Vorbehandlungsanlagen in der Oberflächentechnik (z.B. Automobillackierung), ein oder mehrere kathodische und/oder anodische Tauchlackieranlagen; ein oder mehrere Spritzkabinen mit Nassabscheidung des Overspray.

Anstelle der regelmäßigen Probennahmen können, je nach Erfordernis, ein oder mehrere Messfühler in Form von Biosensoren in die Behälter oder Rohrleitungen der genannten Prozessbehälter, Spülbehälter und/oder Vorlagebehälter eingebaut werden. Diese Biosensoren können den Aufwuchs von Biofilmen von Beginn an erfassen und überwachen.

Abhängig von den Erfordernissen der jeweiligen Teilbereiche der Behandlungsanlage können individuelle, auf das jeweilige System abgestimmte, Grenzwerte eingestellt werden, die je nach Biofilmdicke zu Alarmmeldungen, Bioziddosierungen oder Änderungen z.B. der Dosiermenge bei kontinuierlichen Bioziddosierungen führen.

Gemäß einer günstigen Ausgestaltung kann wenigstens einer der Teilbereiche als Wasseraufbereitungseinrichtung und/oder Wassertank und/oder Vorbehandlungsbecken und/oder Tauchbad und/oder Sprühtunnel und/oder Rohrleitung zur Führung des Fluids und/oder eine Transportstrecke des wenigstens einen Bauteils ausgebildet sein.

Besonders in nassen oder feuchten Bereichen der Behandlungsanlage ist mit einer Biofilmbildung zu rechnen, die letztlich zur Verschleppung von Keimen in die Prozessmedien hinein führt.

Gemäß einer günstigen Ausgestaltung kann der wenigstens eine Messfühler in einem Abschnitt des wenigstens einen Teilbereichs angeordnet sein, in dem eine höhere als durchschnittliche Wahrscheinlichkeit eines frühzeitigen und/oder verstärkten Auftretens von biologischen Verunreinigungsschichten vorliegt. Damit ist eine frühzeitige Detektion und ein frühzeitiger Eingriff in Form einer Biozidbehandlung möglich. Durch die frühe Erkennung kann eine Biozidbehandlung in der Behandlungsanlage gut auf die darin ablaufenden Prozesse und die Auslastung der Behandlungsanlage abgestimmt werden.

Gemäß einer günstigen Ausgestaltung kann der wenigstens eine Messfühler mit einer Steuerungs- und/oder Regelungseinheit verbunden sein, die anhand eines Messwerts, der eine biologische Verunreinigungsschicht anzeigt, durch den wenigstens einen Messfühler im wenigstens einen Teilbereich abhängig von einem vorgegebenen Grenzwert eine Alarmmeldung ausgeben und/oder eine Bioziddosierung in den wenigstens einen Teilbereich hinein durchführen und/oder bei kontinuierlicher Bioziddosierung eine Anpassung einer Bioziddosierung durchführen kann. Ein biozidsparender und zeitlich günstiger Reinigungsablauf kann erfolgen.

Gemäß einer günstigen Ausgestaltung kann der wenigstens eine Messfühler in einem Wandbereich des wenigstens einen Teilbereichs angeordnet sein, welcher mit dem Fluid nicht direkt in Kontakt ist. Ein Biofilm kann relativ ungestört aufwachsen und früh erkannt werden.

Der Messfühler unterliegt günstigerweise denselben mikrobiologischen Bedingungen wie der Teilbereich.

Ebenso ist eine unmittelbare Erfassung der Wirksamkeit einer Biozidbehandlung durch Entfernung oder Verkleinerung des aufgewachsenen Biofilms auf dem Messfühler möglich. Hierzu kann der Messfühler vorteilhaft in der Wand des Teilbereichs eingelassen sein.

Gemäß einer günstigen Ausgestaltung kann der wenigstens eine Messfühler eine selektive Sensitivität für die biologische Verunreinigungsschicht, insbesondere für Biofilme, aufweisen. Fehlmessungen können verringert werden.

### Zeichnung

Weitere Vorteile ergeben sich aus der folgenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen, solange die resultierenden Kombinationen unter dem Schutzumfang der Ansprüche fallen.

Es zeigen beispielhaft:
- Fig. 1: ein Ausführungsbeispiel der Erfindung, mit einer Behandlungsanlage;
- Fig. 2: ein Flussdiagramm eines Verfahrens nach einem Ausführungsbeispiel der Erfindung.

### Ausführungsformen der Erfindung

Die Figuren zeigen lediglich Beispiele und sind nicht beschränkend zu verstehen.

Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

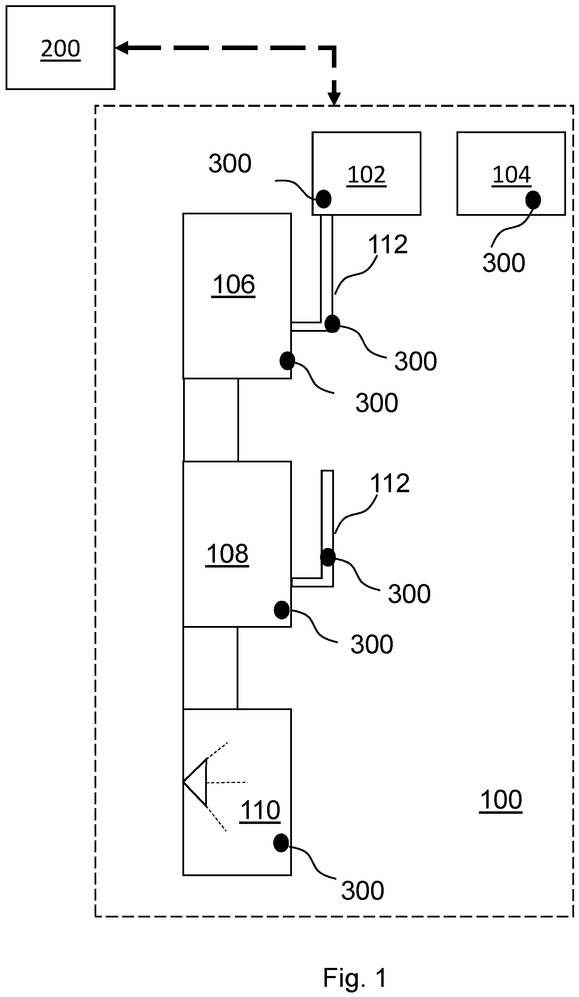

Figur 1 illustriert schematisch ein Ausführungsbeispiel einer Behandlungsanlage 100 mit Teilbereichen 102, 104, 106, 108, 110, 112 zur Durchführung des erfindungsgemäßen Verfahrens.

Teilbereiche 102, 104, 106, 108, 110, 112 der mit dem Verfahren betriebenen Behandlungsanlage 100 können beispielsweise umfassen Befeuchter von Zu- und Umluftanlagen von z.B. Spritzkabinen, Hallenbelüftung, etc., Frischwassersysteme; VE-Wassersysteme mit vollensalztem Wasser, hier vor allem ein oder mehrere Ionentauscher und Pufferbehälter, ein oder mehrere Membrananlagen zur Frischwasseraufbereitung und/oder Abwasseraufbereitung, ein oder mehrere Vorbehandlungsanlagen in der Oberflächentechnik (z.B. Automobillackierung), ein oder mehrere kathodische und/oder anodische Tauchlackieranlagen; ein oder mehrere Spritzkabinen mit Nassabscheidung des Overspray.

Die Teilbereiche 102, 104, 106, 108, 110, 112 sind im gezeigten Ausführungsbeispiel beispielsweise eine Wasseraufbereitungseinrichtung 102, ein Wassertank 104, ein Vorbehandlungsbecken 106, ein Tauchbad 108 sowie ein Sprühtunnel 110 und beispielhaft eingezeichnete Rohrleitungen 112 zur Führung des Fluids sowie Transportstrecken zum Transport von Bauteilen zwischen den Teilbereichen. In der Behandlungsanlage 100 können beispielsweise Fahrzeugkarossen lackiert werden.

Beispielhaft sind in jedem der Teilbereiche 102, 104, 106, 108, 110, 112 jeweils ein Messfühler 300 montiert, der wenigstens eine biologische Komponente zum Nachweis wenigstens einer biologischen Verunreinigungsschicht in dem wenigstens einen Teilbereich 102, 104, 106, 108, 110, 112 aufweist.

Günstigerweise sind die Messfühler 300 in einem Abschnitt der Teilbereiche 102, 104, 106, 108, 110, 112 angeordnet, in dem eine höhere als durchschnittliche Wahrscheinlichkeit eines frühzeitigen und/oder verstärkten Auftretens von biologischen Verunreinigungsschichten vorliegt.

Die Messfühler 300 sind mit einer Steuerungs- und/oder Regelungseinheit 200 verbunden, die anhand jeweils von Messwerten einer biologischen Verunreinigungsschicht in Form eines Biofilms durch die Messfühler 300 in den Teilbereichen 102, 104, 106, 108, 110, 112 abhängig von einem jeweils vorgegebenen Grenzwert eine Alarmmeldung ausgibt und/oder eine Bioziddosierung in den betreffenden Teilbereichen 102, 104, 106, 108, 110, 112 hinein durchführt und/oder bei kontinuierlicher Bioziddosierung eine Anpassung einer Bioziddosierung durchführt.

Vorteilhaft ist die Steuerungs- und/oder Regelungseinheit 200 mit einem Rechner verbunden, der ein Computerprogrammprodukt zur Durchführung des erfindungsgemäßen Verfahrens zum Betreiben einer Behandlungsanlage 100 ausführt, umfassend wenigstens ein computerlesbares Speichermedium mit darauf gespeicherten Programmcodeanweisungen, wobei die von einer Datenverarbeitungsanlage ausführbaren Programmcodeanweisungen bewirken, dass ein Grenzwert einer biologischen Verunreinigungsschicht zum Nachweis durch den wenigstens einen Messfühler 300 in wenigstens einem Teilbereich 102, 104, 106, 108, 110, 112 abhängig von jeweiligen Prozesserfordernissen in dem wenigstens einen Teilbereich 102, 104, 106, 108, 110, 112 eingestellt wird;
dass kontinuierlich oder in vorgegebenen Zeitintervallen geprüft wird, ob ein Messwert des wenigstens einen Messfühlers 300 im wenigstens einen Teilbereich 102, 104, 106, 108, 110, 112 den vorgegebenen Grenzwert verletzt;
dass bei einer Verletzung des Grenzwerts für den entsprechenden Teilbereich 102, 104, 106, 108, 110, 112 eine Aktion ausgelöst wird, insbesondere dass eine Alarmmeldung ausgegeben wird und/oder eine Behandlung des entsprechenden Teilbereichs 102, 104, 106, 108, 110, 112 mit einem Biozid, oder, bei kontinuierlicher Zudosierung eines Biozids in einen oder mehreren der Teilbereiche 102, 104, 106, 108, 110, 112 die Bioziddosierung angepasst wird;
dass nach Abschluss der Aktion die kontinuierliche Überwachung des wenigstens einen Teilbereichs 102, 104, 106, 108, 110, 112 fortgesetzt wird;
dass, falls der Grenzwert nicht verletzt wird, die kontinuierliche Überwachung des wenigstens einen Teilbereichs 102, 104, 106, 108, 110, 112 fortgesetzt wird.

Günstigerweise sind die Messfühler 300 jeweils in einem Wandbereich der Teilbereiche 102, 104, 106, 108, 110, 112 angeordnet, welcher mit dem Fluid nicht direkt in Kontakt ist. Die Messfühler 300 unterliegen denselben mikrobiologischen Bedingungen wie der Teilbereich 102, 104, 106, 108, 110, 112.

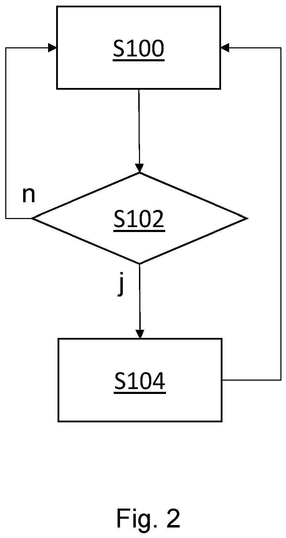

In Figur 2 ist ein Flussdiagramm dargestellt, welches das Verfahren zum Betreiben einer Behandlungsanlage 100 mit wenigstens einem Teilbereich 102, 104, 106, 108, 110, 112, in dem Fluid zur Behandlung von Bauteilen aufbewahrt wird, und/oder eine Behandlung von Bauteilen, insbesondere mit dem Fluid, durchgeführt wird, und/oder ein Fluid und/oder ein Bauteil transportiert wird, beschreibt, wobei wenigstens einer der Teilbereiche 102, 104, 106, 108, 110, 112 durch wenigstens einen Messfühler 300 überwacht wird, der wenigstens eine biologische Komponente zum Nachweis wenigstens einer biologischen Verunreinigungsschicht, insbesondere eines Biofilms, in dem wenigstens einen Teilbereich 102, 104, 106, 108, 110, 112 aufweist.

In Schritt S100 erfolgt eine kontinuierliche Überwachung des wenigstens einen Teilbereichs 102, 104, 106, 108, 110, 112 durch den wenigstens einen Messfühler 300.

Dabei wird ein Grenzwert einer biologischen Verunreinigungsschicht zum Nachweis durch den wenigstens einen Messfühler 300 im wenigstens einen Teilbereich 102, 104, 106, 108, 110, 112 abhängig von jeweiligen Prozesserfordernissen in dem wenigstens einen Teilbereich 102, 104, 106, 108, 110, 112 eingestellt.

In Schritt S102 wird kontinuierlich oder in vorgegebenen Zeitintervallen geprüft, ob ein Messwert des wenigstens einen Messfühlers 300 im wenigstens einen Teilbereich 102, 104, 106, 108, 110, 112 den vorgegebenen Grenzwert verletzt. Ein Grenzwert kann situationsabhängig verletzt sein, wenn dieser erreicht wird, unterschritten wird oder überschritten wird. Dies kann je nach Teilbereich 102, 104, 106, 108, 110, 112, eingesetztem Behandlungsverfahren und dergleichen gewählt werden.

Falls der Grenzwert verletzt wird ("j" im Flussdiagramm), wird in Schritt S104 für den entsprechenden Teilbereich 102, 104, 106, 108, 110, 112 eine Alarmmeldung ausgegeben. Alternativ oder zusätzlich erfolgt eine Behandlung des entsprechenden Teilbereichs 102, 104, 106, 108, 110, 112 mit einem Biozid, oder, bei kontinuierlicher Zudosierung eines Biozids in einen oder mehreren der Teilbereiche 102, 104, 106, 108, 110, 112, wird die Bioziddosierung angepasst.

Nach Abschluss von Schritt S104 wird die kontinuierliche Überwachung des wenigstens einen Teilbereichs 102, 104, 106, 108, 110, 112 in Schritt S100 fortgesetzt.

Falls der Grenzwert nicht verletzt wird ("n" im Flussdiagramm), wird die kontinuierliche Überwachung des wenigstens einen Teilbereichs 102, 104, 106, 108, 110, 112 in Schritt S100 fortgesetzt.

## Patentansprüche

1. Verfahren zum Betreiben einer Behandlungsanlage (100) mit wenigstens einem Teilbereich (102, 104, 106, 108, 110, 112), in dem Fluid zur Behandlung von Bauteilen aufbewahrt wird, und/oder eine Behandlung von Bauteilen, insbesondere mit dem Fluid, durchgeführt wird, und/oder ein Fluid und/oder ein Bauteil transportiert wird, wobei wenigstens einer der Teilbereiche (102, 104, 106, 108, 110, 112) durch wenigstens einen Messfühler (300) überwacht wird, der wenigstens eine biologische Komponente zum Nachweis wenigstens einer biologischen Verunreinigungsschicht in Form eines Biofilms als Vorstadium einer Verkeimung in dem wenigstens einen Teilbereich (102, 104, 106, 108, 110, 112) aufweist, wobei eine Erfassung und Überwachung der biologischen Verunreinigungsschicht von Beginn eines Aufwuchses der biologischen Verunreinigungsschicht auf dem Messfühler (300) an erfolgt.

2. Verfahren nach Anspruch 1, wobei eine Überwachung des wenigstens einen Teilbereichs (102, 104, 106, 108, 110, 112) durch den wenigstens einen Messfühler (300) kontinuierlich durchgeführt wird.

3. Verfahren nach Anspruch 1 oder 2, wobei ein Grenzwert einer biologischen Verunreinigungsschicht zum Nachweis durch den wenigstens einen Messfühler (300) im wenigstens einen Teilbereich (102, 104, 106, 108, 110, 112) abhängig von jeweiligen Prozesserfordernissen in dem wenigstens einen Teilbereich (102, 104, 106, 108, 110, 112) eingestellt wird.

4. Verfahren nach einem der vorhergehenden Ansprüche, wobei bei Erfassung eines Messwerts einer biologischen Verunreinigungsschicht durch den wenigstens einen Messfühler (300) im wenigstens einen Teilbereich (102, 104, 106, 108, 110, 112) abhängig von einem vorgegebenen Grenzwert eine Reinigungssequenz in dem wenigstens einen Teilbereich (102, 104, 106, 108, 110, 112) durchgeführt wird.

5. Verfahren nach einem der vorhergehenden Ansprüche, wobei bei Erfassung eines Messwerts einer biologischen Verunreinigungsschicht durch den wenigstens einen Messfühler (300) im wenigstens einen Teilbereich (102, 104, 106, 108, 110, 112) abhängig von einem vorgegebenen Grenzwert eine Alarmmeldung erfolgt und/oder eine Bioziddosierung in den wenigstens einen Teilbereich (102, 104, 106, 108, 110, 112) hinein erfolgt und/oder bei kontinuierlicher Bioziddosierung in den Teilereich (102, 104, 106, 108, 110, 112) eine Anpassung einer Bioziddosierung erfolgt.

6. Verfahren nach einem der vorhergehenden Ansprüche, wobei eine Position des wenigstens einen Messfühlers (300) in dem wenigstens einen Teilbereich (102, 104, 106, 108, 110, 112) nach einer Wahrscheinlichkeit eines frühzeitigen und/oder verstärkten Auftretens von biologischen Verunreinigungsschichten ausgewählt wird.

7. Verfahren nach einem der vorhergehenden Ansprüche, wobei eine Reinigungssequenz bei einer Behandlungsanlage (100) mit mehreren Teilbereichen (102, 104, 106) jeweils für jeden der Teilbereiche (102, 104, 106, 108, 110, 112) einzeln durchgeführt wird.

8. Behandlungsanlage (100) mit wenigstens einem Teilbereich (102, 104, 106, 108, 110, 112) zur Durchführung des Verfahrens nach einem der vorhergehenden Ansprüche, wobei wenigstens ein Teilbereich (102, 104, 106, 108, 110, 112) vorgesehen ist, in dem Fluid zur Behandlung von Bauteilen aufbewahrt wird, und/oder eine Behandlung von Bauteilen, insbesondere mit dem Fluid, durchgeführt wird, und/oder ein Fluid und/oder ein Bauteil transportiert wird, wobei wenigstens einer der Teilbereiche (102, 104, 106, 108, 110, 112) wenigstens einen Messfühler (300) aufweist, der wenigstens eine biologische Komponente zum Nachweis wenigstens einer biologischen Verunreinigungsschicht in Form eines Biofilms als Vorstadium einer Verkeimung in dem wenigstens einen Teilbereich (102, 104, 106, 108, 110, 112) aufweist,
wobei die biologische Verunreinigungsschicht von Beginn eines Aufwuchses der biologischen Verunreinigungsschicht auf dem Messfühler (300) an erfassbar und überwachbar ist.

9. Behandlungsanlage nach Anspruch 8, wobei wenigstens einer der Teilbereiche (102, 104, 106, 108, 110, 112) als Wasseraufbereitungseinrichtung (102) und/oder Wassertank (104) und/oder Vorbehandlungsbecken (106) und/oder Tauchbad (108) und/oder Sprühtunnel (110) und/oder Rohrleitung (112) zur Führung des Fluids ausgebildet ist.

10. Behandlungsanlage nach Anspruch 8 oder 9, wobei der wenigstens eine Messfühler (300) in einem Abschnitt des wenigstens einen Teilbereichs (102, 104, 106, 108, 110, 112) angeordnet ist, in dem eine höhere als durchschnittliche Wahrscheinlichkeit eines frühzeitigen und/oder verstärkten Auftretens von biologischen Verunreinigungsschichten vorliegt.

11. Behandlungsanlage nach einem der Ansprüche 8 bis 10, wobei der wenigstens eine Messfühler (300) mit einer Steuerungs- und/oder Regelungseinheit (200) verbunden ist, die anhand eines Messwerts einer biologischen Verunreinigungsschicht durch den wenigstens einen Messfühler (300) im wenigstens einen Teilbereich (102, 104, 106, 108, 110, 112) abhängig von einem vorgegebenen Grenzwert eine Alarmmeldung ausgibt und/oder eine Bioziddosierung in den wenigstens einen Teilbereich (102, 104, 106, 108, 110, 112) hinein durchführt und/oder bei kontinuierlicher Bioziddosierung eine Anpassung einer Bioziddosierung durchführt.

12. Behandlungsanlage nach einem der Ansprüche 8 bis 11, wobei der wenigstens eine Messfühler (300) in einem Wandbereich des wenigstens einen Teilbereichs (102, 104, 106, 108, 110, 112) angeordnet ist, welcher mit dem Fluid nicht direkt in Kontakt ist.

13. Behandlungsanlage nach einem der Ansprüche 8 bis 12, wobei der wenigstens eine Messfühler (300) eine selektive Sensitivität für die biologische Verunreinigungsschicht, insbesondere für Biofilme, aufweist.

## Claims

1. Method for operating a treatment installation (100) having at least one subregion (102, 104, 106, 108, 110, 112) in which fluid for treating components is stored and/or treatment of components, in particular with the fluid, is carried out and/or a fluid and/or a component is transported, wherein at least one of the subregions (102, 104, 106, 108, 110, 112) is monitored by at least one sensor (300) which has at least one biological component for detecting at least one biological contamination layer in the form of a biofilm as an initial stage of contamination in the at least one subregion (102, 104, 106, 108, 110, 112), wherein sensing and monitoring of the biological contamination layer is carried out from the beginning of growth of the biological contamination layer on the sensor (300).

2. Method according to Claim 1, wherein monitoring of the at least one subregion (102, 104, 106, 108, 110, 112) is continuously carried out by the at least one sensor (300).

3. Method according to Claim 1 or 2, wherein a limit value of a biological contamination layer for detection by the at least one sensor (300) in the at least one subregion (102, 104, 106, 108, 110, 112) is set depending on respective process requirements in the at least one subregion (102, 104, 106, 108, 110, 112).

4. Method according to any of the preceding claims, wherein, when a measurement value of a biological contamination layer is sensed by the at least one sensor (300) in the at least one subregion (102, 104, 106, 108, 110, 112), a cleaning sequence is carried out in the at least one subregion (102, 104, 106, 108, 110, 112), depending on a specified limit value.

5. Method according to any of the preceding claims, wherein, when a measurement value of a biological contamination layer is sensed by the at least one sensor (300) in the at least one subregion (102, 104, 106, 108, 110, 112), an alarm message is issued and/or biocide is metered into the at least one subregion (102, 104, 106, 108, 110, 112) and/or biocide metering is adjusted in the event of continuous metering of biocide into the subregion (102, 104, 106, 108, 110, 112), depending on a specified limit value.

6. Method according to any of the preceding claims, wherein a position of the at least one sensor (300) in the at least one subregion (102, 104, 106, 108, 110, 112) is selected according to a probability of early and/or increased occurrence of biological contamination layers.

7. Method according to any of the preceding claims, wherein a cleaning sequence in a treatment installation (100) having several subregions (102, 104, 106) is carried out individually for each of the subregions (102, 104, 106, 108, 110, 112).

8. Treatment installation (100) having at least one subregion (102, 104, 106, 108, 110, 112) for carrying out the method according to any of the preceding claims, wherein at least one subregion (102, 104, 106, 108, 110, 112) is provided, in which fluid for treating components is stored and/or treatment of components, in particular with the fluid, is carried out and/or a fluid and/or a component is transported, wherein at least one of the subregions (102, 104, 106, 108, 110, 112) has at least one sensor (300) which has at least one biological component for detecting at least one biological contamination layer in the form of a biofilm as an initial stage of contamination in the at least one subregion (102, 104, 106, 108, 110, 112),
wherein the biological contamination layer can be sensed and monitored from the beginning of growth of the biological contamination layer on the sensor (300).

9. Treatment installation according to Claim 8, wherein at least one of the subregions (102, 104, 106, 108, 110, 112) is in the form of a water treatment device (102) and/or water tank (104) and/or pretreatment basin (106) and/or immersion bath (108) and/or spray tunnel (110) and/or pipeline (112) for guiding the fluid.

10. Treatment installation according to Claim 8 or 9, wherein the at least one sensor (300) is arranged in a portion of the at least one subregion (102, 104, 106, 108, 110, 112) in which there is a higher than average probability of early and/or increased occurrence of biological contamination layers.

11. Treatment installation according to any of Claims 8 to 10, wherein the at least one sensor (300) is connected to an open-loop and/or closed-loop control unit (200), which emits an alarm message and/or meters biocide into the at least one subregion (102, 104, 106, 108, 110, 112) and/or adjusts biocide metering in the event of continuous metering of biocide based on a measurement value of a biological contamination layer by the at least one sensor (300) in at least one subregion (102, 104, 106, 108, 110, 112), depending on a specified limit value.

12. Treatment installation according to any of Claims 8 to 11, wherein the at least one sensor (300) is arranged in a wall region of the at least one subregion (102, 104, 106, 108, 110, 112) which is not in direct contact with the fluid.

13. Treatment installation according to any of Claims 8 to 12, wherein the at least one sensor (300) has selective sensitivity for the biological contamination layer, in particular for biofilms.

## Revendications

1. Procédé d'exploitation d'une installation de traitement (100) avec au moins une zone partielle (102, 104, 106, 108, 110, 112), dans laquelle un fluide est stocké pour le traitement de composants, et/ou un traitement de composants est exécuté, notamment avec le fluide, et/ou un fluide et/ou un composant est transporté, au moins l'une des zones partielles (102, 104, 106, 108, 110, 112) étant surveillée par au moins un capteur de mesure (300), qui présente au moins un composant biologique pour l'identification d'au moins une couche de contamination biologique sous la forme d'un biofilm en tant que stade préliminaire d'une prolifération de germes dans l'au moins une zone partielle (102, 104, 106, 108, 110, 112), une détection et une surveillance de la couche de contamination biologique étant effectuées dès le début d'une croissance de la couche de contamination biologique sur le capteur de mesure (300).

2. Procédé selon la revendication 1, dans lequel une surveillance de l'au moins une zone partielle (102, 104, 106, 108, 110, 112) est exécutée en continu par l'au moins un capteur de mesure (300).

3. Procédé selon la revendication 1 ou 2, dans lequel une valeur limite d'une couche de contamination biologique pour l'identification par l'au moins un capteur de mesure (300) dans l'au moins une zone partielle (102, 104, 106, 108, 110, 112) est réglée en fonction d'exigences respectives du processus dans l'au moins une zone partielle (102, 104, 106, 108, 110, 112).

4. Procédé selon l'une quelconque des revendications précédentes, dans lequel, lors de la détection d'une valeur de mesure d'une couche de contamination biologique par l'au moins un capteur de mesure (300) dans l'au moins une zone partielle (102, 104, 106, 108, 110, 112), une séquence de nettoyage est exécutée dans l'au moins une zone partielle (102, 104, 106, 108, 110, 112) en fonction d'une valeur limite prédéfinie.

5. Procédé selon l'une quelconque des revendications précédentes, dans lequel, en cas de détection d'une valeur de mesure d'une couche de contamination biologique par l'au moins un capteur de mesure (300) dans l'au moins une zone partielle (102, 104, 106, 108, 110, 112) en fonction d'une valeur limite prédéfinie, un message d'alarme est effectué et/ou un dosage de biocide est effectué dans l'au moins une zone partielle (102, 104, 106, 108, 110, 112) et/ou, en cas de dosage continu de biocide dans la zone partielle (102, 104, 106, 108, 110, 112), une adaptation d'un dosage de biocide est effectuée.

6. Procédé selon l'une quelconque des revendications précédentes, dans lequel une position de l'au moins un capteur de mesure (300) dans l'au moins une zone partielle (102, 104, 106, 108, 110, 112) est sélectionnée selon une probabilité d'apparition précoce et/ou accrue de couches de contamination biologique.

7. Procédé selon l'une quelconque des revendications précédentes, dans lequel, dans une installation de traitement (100) avec plusieurs zones partielles (102, 104, 106), une séquence de nettoyage est exécutée individuellement pour chacune des zones partielles (102, 104, 106, 108, 110, 112).

8. Installation de traitement (100) avec au moins une zone partielle (102, 104, 106, 108, 110, 112) pour l'exécution du procédé selon l'une quelconque des revendications précédentes, dans laquelle au moins une zone partielle (102, 104, 106, 108, 110, 112) est prévue, dans laquelle un fluide est stocké pour le traitement de composants, et/ou un traitement de composants est exécuté, notamment avec le fluide, et/ou un fluide et/ou un composant est transporté, au moins l'une des zones partielles (102, 104, 106, 108, 110, 112) présentant au moins un capteur de mesure (300) qui présente au moins un composant biologique pour l'identification d'au moins une couche de contamination biologique sous la forme d'un biofilm en tant que stade préliminaire d'une prolifération de germes dans l'au moins une zone partielle (102, 104, 106, 108, 110, 112),
la couche de contamination biologique pouvant être détectée et surveillée dès le début d'une croissance de la couche de contamination biologique sur le capteur de mesure (300).

9. Installation de traitement selon la revendication 8, dans laquelle au moins l'une des zones partielles (102, 104, 106, 108, 110, 112) est réalisée sous la forme d'un dispositif de conditionnement d'eau (102) et/ou d'un réservoir d'eau (104) et/ou d'un bassin de prétraitement (106) et/ou d'un bain d'immersion (108) et/ou d'un tunnel de pulvérisation (110) et/ou d'une tuyauterie (112) pour le guidage du fluide.

10. Installation de traitement selon la revendication 8 ou 9, dans laquelle l'au moins un capteur de mesure (300) est agencé dans une section de l'au moins une zone partielle (102, 104, 106, 108, 110, 112) dans laquelle il existe une probabilité plus élevée que moyenne d'apparition précoce et/ou accrue de couches de contamination biologique.

11. Installation de traitement selon l'une quelconque des revendications 8 à 10, dans laquelle l'au moins un capteur de mesure (300) est relié à une unité de commande et/ou de régulation (200) qui, à l'aide d'une valeur de mesure d'une couche de contamination biologique par l'au moins un capteur de mesure (300) dans au moins une zone partielle (102, 104, 106, 108, 110, 112) en fonction d'une valeur limite prédéterminée émet un message d'alarme et/ou exécute un dosage de biocide dans l'au moins une zone partielle (102, 104, 106, 108, 110, 112) et/ou, en cas de dosage continu de biocide, exécute une adaptation d'un dosage de biocide.

12. Installation de traitement selon l'une quelconque des revendications 8 à 11, dans laquelle l'au moins un capteur de mesure (300) est agencé dans une zone de paroi de l'au moins une zone partielle (102, 104, 106, 108, 110, 112) qui n'est pas en contact direct avec le fluide.

13. Installation de traitement selon l'une quelconque des revendications 8 à 12, dans laquelle l'au moins un capteur de mesure (300) présente une sensibilité sélective à la couche de contamination biologique, notamment aux biofilms.
